# EUROPÄISCHE PATENTSCHRIFT

(11) **EP 1 526 904 B1**
(45) Veröffentlichungstag und Bekanntmachung des Hinweises auf die Patenterteilung: **02.08.2006**
(21) Anmeldenummer: 03765100.7
(22) Anmeldetag: 23.07.2003
(51) Int. Cl.: B01D 3/32, B01D 3/14, C07C 7/04, C07B 41/00, C07D 301/12

(54) **VERFAHREN ZUR KONTINUIERLICH BETRIEBENEN REINDESTILLATION VON OXIRANEN, SPEZIELL VON PROPYLENOXID**
METHOD FOR CONTINUOUSLY OPERATED PURE DISTILLATION OF OXIRANES, ESPECIALLY PROPYLENE OXIDE
PROCEDE DE DISTILLATION PURE OPEREE EN CONTINU D'OXIRANES, EN PARTICULIER D'OXYDE DE PROPYLENE

(30) Priorität: 23.07.2002 DE 10233387
(43) Veröffentlichungstag der Anmeldung: 04.05.2005
(73) Patentinhaber: BASF Aktiengesellschaft, 67056 Ludwigshafen (DE)
(72) Erfinder: BASSLER, Peter, 68519 Viernheim (DE); GÖBBEL, Hans-Georg, 67169 Kallstadt (DE); TELES, Joaquim, Henrique, 67166 Otterstadt (DE); RUDOLF, Peter, 68526 Ladenburg (DE)
(74) Vertreter: Isenbruck, Günter
(86) Internationale Anmeldenummer: PCT/EP2003/008043
(87) Internationale Veröffentlichungsnummer: WO 2004/009204

(56) Entgegenhaltungen:
- EP-A- 0 122 367
- EP-A- 1 151 781
- WO-A-00/07965
- WO-A-02/40434

## Beschreibung

Die Erfindung betrifft ein kontinuierlich betriebenes Verfahren zur Reindestillation des bei der vorzugsweise koppelproduktfreien Oxiransynthese durch Umsetzung eines Hydroperoxids mit einer organischen Verbindung entstehenden Oxirans. Das dabei anfallende Rohprodukt wird in einer Trennwandkolonne in Form thermisch gekoppelten Kolonnen in eine eine Leicht-, Mittel- und Hochsiederfraktion aufgetrennt und das Oxiran als Mittelsieder an der Seitenabzugsstelle der Kolonne entnommen. Insbesondere betrifft die Erfindung die kontinuierlich betriebene Reindestillation von Propylenoxid aus der Umsetzung von Propylen mit Wasserstoffperoxid.

Nach den gängigen Verfahren des Standes der Technik können Oxirane durch Umsetzung geeigneter organischer Verbindungen mit Hydroperoxiden in einstufigen oder mehrstufigen Umsetzungen hergestellt werden.

Im Vergleich zum einstufigen Verfahren kann in mehrstufigen Verfahren der Überschuss an umzusetzender organischer Verbindung relativ klein gehalten werden. Dadurch wird die Selektivität bezüglich der Oxiranbildung erhöht.

Beispielsweise sieht das in der WO 00/07965 beschriebene mehrstufige Verfahren vor, dass die Umsetzung der organischen Verbindung mit einem Hydroperoxid wenigstens die Schritte (i) bis (iii) umfasst:
(i) Umsetzung des Hydroperoxids mit der organischen Verbindung unter Erhalt eines Produktgemisches, umfassend die umgesetzte organische Verbindung und nicht umgesetztes Hydroperoxid,
(ii) Abtrennung des nicht umgesetzten Hydroperoxids aus der aus Stufe (i) resultierenden Mischung,
(iii) Umsetzung des abgetrennten Hydroperoxids aus Stufe (ii) mit der organischen Verbindung.

Demgemäss findet die Umsetzung der organischen Verbindung mit dem Hydroperoxid in mindestens zwei Stufen (i) und (iii) statt, wobei das in Stufe (ii) abgetrennte Hydroperoxid erneut in die Reaktion eingesetzt wird.

Vorzugsweise erfolgen dabei die Umsetzungen in den Stufen (i) und (iii) in zwei getrennten Reaktoren, vorzugsweise Festbettreaktoren, wobei vorzugsweise die Umsetzung der Stufe (i) in einem isothermen und die Umsetzung der Stufe (iii) in einem adiabatischen Reaktor stattfindet.

Allgemein kann dieses Verfahren zur Umsetzung von Alkenen mit Hydroperoxiden zu Oxiranen verwendet werden. Vorzugsweise wird bei dieser Sequenz als Hydroperoxid Wasserstoffperoxid verwendet sowie die organische Verbindung während der Reaktion in Kontakt mit einem heterogenen Katalysator gebracht.

Wegen der hohen Selektivität der Reaktion wird diese Herstellmethode auch als koppelproduktfreie Oxiransynthese bezeichnet.

Insbesondere kann obiges Verfahren zur Herstellung von Proplylenoxid aus Propylen und Wasserstoffperoxid verwendet werden. Hierbei erreicht der Wasserstoffperoxid-Umsatz in Stufe (i) ca. 85 % bis 90 % und in Stufe (iii) ca. 95 % bezogen auf Stufe (ii). In der Summe kann über beide Stufen ein Wasserstoffperoxidumsatz von ca. 99 % bei einer Propylenoxid-Selektivität von ca. 94 - 95 % erreicht werden.

Das bei dieser Oxiransynthese und der Aufarbeitung erhaltene rohe Oxiran enthält noch Verunreinigungen. Es fällt im Allgemeinen in einer Konzentration von ca. 95 bis 99 % an. Um als Ausgangsprodukt für spezielle organische Synthesen geeignet zu sein, z. B. bei der Polyurethanherstellung, ist häufig eine höhere Konzentration von mindestens 99,9 % erforderlich. Es muss somit einem Reinigungsschritt unterzogen werden.

Die nach dem Stand der Technik durchgeführte Reindestillation erfolgt in konventionellen Kolonnen mit Seitenabzug oder Kolonnen in Reihenschaltung. Hierzu ist ein relativ hoher apparativer und energetischer Aufwand notwendig, da das Oxiran bis zur vorstehend genannten hohen Reinheit mehrmals destilliert werden muss.

Es war Aufgabe der vorliegenden Erfindung, die Reindestillation der bei der Umsetzung von geeigneten organischen Verbindungen mit Hydroperoxiden entstehenden Oxirane zu optimieren, insbesondere im Sinne des Energieverbrauchs und der thermischen Belastung. Insbesondere sollte ein kontinuierlich zu betreibendes Verfahren zur Verfügung gestellt werden, welches erlaubt, die vorzugsweise durch mehrstufige Umsetzung erhaltenen Oxirane bei möglichst geringem apparativen und energetischen Aufwand durch Reindestillation in hoher Reinheit zu gewinnen.

Diese Aufgabe konnte durch ein kontinuierlich betriebenes Verfahren zur Reindestillation des bei der vorzugsweise koppelproduktfreien Oxiransynthese durch Umsetzung eines Hydroperoxids mit einer organischen Verbindung entstehenden Oxirans mittels einer Trennwandkolonne in Form thermisch gekoppelten Kolonnen gelöst werden.

Gegenstand der Erfindung ist daher ein kontinuierlich betriebenes Verfahren zur Reindestillation des bei der Oxiransynthese durch Umsetzung eines Hydroperoxids mit einer organischen Verbindung entstehenden Oxirans, dadurch gekennzeichnet, dass das Rohoxiran in einer Trennwandkolonne in eine Leicht-, Mittel- und Hochsiederfraktion aufgetrennt und als Mittelsieder am Seitenabzug entnommen wird.

Nach dem erfindungsgemäßen Verfahren kann das Oxiran in hoher Reinheit von vorzugsweise mindestens 99,9 % unter schonenden Destillationsbedingungen gewonnen werden. Es kann von Verunreinigungen unter geringer thermischer Belastung abgetrennt werden, da aufgrund der einmaligen Verdampfung nur kurze Verweilzeiten in der Trennwandkolonne notwendig sind. Dies ist für die Destillation der hoch reaktiven und thermisch labilen Oxirane außerordentlich vorteilhaft. Im Vergleich zu den beim Stand der Technik geschilderten Destillationsverfahren führt deshalb das neue erfindungsgemäße Verfahren zu einem reduzierten apparativen und energetischen Aufwand bei gleichzeitig verbesserter Produktqualität. Für die industrielle Anwendung ist dies außerordentlich vorteilhaft.

Vorzugsweise wird das Verfahren zur Destillation von Propylenoxid eingesetzt, das durch mehrstufige Umsetzung von Propylen mit Wasserstoffperoxid hergestellt wird.

Destillationskolonnen mit Seitenabzügen und Trennwand, im Folgenden auch als Trennwandkolonnen bezeichnet, sind bereits bekannt. Sie stellen eine Weiterentwicklung von Destillationskolonnen dar, die nur den Seitenabzug, jedoch keine Trennwand besitzen. Die Anwendungsmöglichkeit des zuletzt genannten konventionellen Kolonnentyps ist aber eingeschränkt, weil die an den Seitenabzugsstellen entnommenen Produkte nie völlig rein sind. Bei Seitenabnahmen im Verstärkungsteil der Kolonne, die üblicherweise in flüssiger Form erfolgen, enthält das Seitenprodukt noch Anteile an leichtsiedenden Komponenten, die über Kopf abgetrennt werden sollen. Bei Seitenabnahmen im Abtriebsteil der Kolonne, die meist dampfförmig erfolgen, weist das Seitenprodukt noch Hochsiederanteile auf. Die Verwendung von konventionellen Seitenabzugskolonnen ist daher auf Fälle begrenzt, in denen verunreinigte Seitenprodukte zulässig sind.

Beim Einbau einer Trennwand in eine solche Kolonne kann die Trennwirkung verbessert werden. Damit ist es möglich, Seitenprodukte in reiner Form zu entnehmen. Im mittleren Bereich oberhalb und unterhalb der Zulaufstelle und der Seitenentnahmestelle ist eine Trennwand angebracht, wobei diese fest verschweißt oder auch nur gesteckt werden kann. Sie dichtet den Entnahmeteil gegenüber dem Zulaufteil ab und unterbindet in diesem Kolonnenteil eine Quervermischung von Flüssigkeits- und Brüdenströmen über den gesamten Kolonnenquerschnitt. Hierdurch verringert sich bei der Auftrennung von Vielstoffgemischen, deren Komponenten ähnliche Siedepunkte besitzen, die Zahl der insgesamt benötigten Destillationskolonnen.

Dieser Kolonnentyp wurde beispielsweise zur Trennung einer Komponentenvorlage aus Methan, Ethan, Propan und Butan verwendet (US 2,471,134), zur Trennung eines Gemisches von Benzol, Toluol und Xylol (US 4,230,533) sowie zur Trennung eines Gemisches aus n-Hexan, n-Heptan und n-Octan (EP 0 122 367).

Auch zur Trennung azeotrop siedender Mischungen können Trennwandkolonnen erfolgreich eingesetzt werden (EP 0 133 510).

Schließlich sind auch Trennwandkolonnen bekannt, in denen chemische Reaktionen unter gleichzeitiger Destillation der Produkte durchgeführt werden können. Als Beispiele werden Veresterungen, Umesterungen, Verseifungen sowie Acetalisierungen genannt (EP 0 126 288).

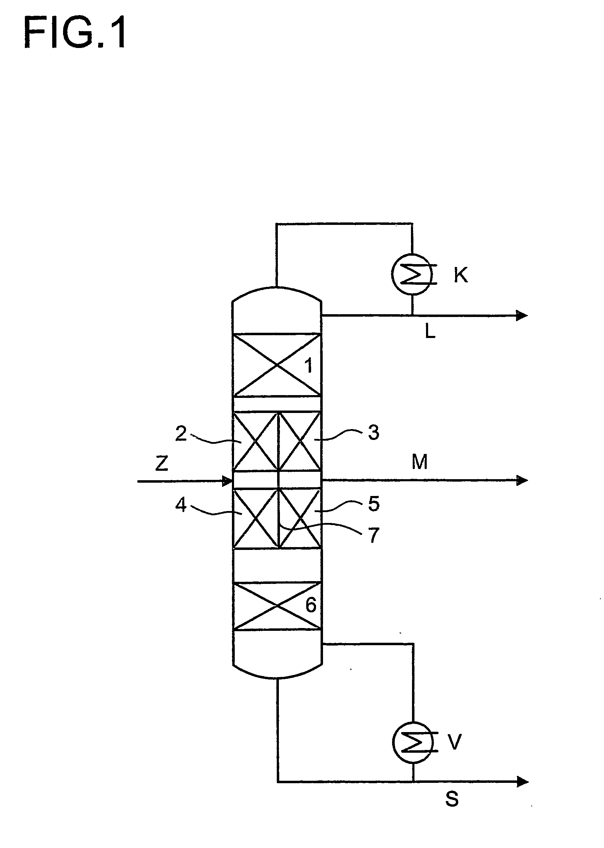

In Figur 1 ist schematisch die Reindestillation des bei der Oxiransynthese entstehenden Oxirans in einer Trennwandkolonne dargestellt. Dabei wird das aus der Oxiransynthese stammende Rohoxiran über den Zulauf Z in die Kolonne eingebracht. In der Kolonne wird besagtes Rohoxiran aufgetrennt in eine Fraktion, die die Leichtsieder L enthält, beispielsweise Zersetzungs- und Spaltprodukte wie Acetaldehyd und Methylformiat, und in eine Hochsiederfraktion S, die beispielsweise Lösungsmittel und Wasser enthält. Das hochreine Oxiran wird am Seitenabzug für Mittelsieder M entnommen.

Zur Entnahme an der Seitenentnahmestelle eignen sich sowohl innenliegende als auch außerhalb der Kolonne angeordnete Auffangräume, in denen die Flüssigkeit oder kondensierender Dampf gesammelt werden kann.

Vorzugsweise werden zur Oxiransynthese das in der WO 00/07965 beschriebene Verfahren sowie die Vorrichtung zur Durchführung des Verfahrens verwendet. Die Vorrichtung besteht aus einem isothermen Festbettreaktor, einer Abtrennvorrichtung und einem adiabatischen Festbettreaktor.

Demzufolge ist das erfindungsgemäße Verfahren zur vorzugsweise kontinuierlichen Reindestillation eines Oxirans geeignet, das hergestellt wird durch ein Verfahren umfassend wenigstens die Schritte (i) bis (iii) wie vorstehend definiert.

Zur Durchführung des erfindungsgemäßen Verfahrens können übliche Trennwandkolonnen mit einem oder zwei Seitenabzügen, etwa wie sie beim Stand der Technik genannt sind, verwendet werden.

Eine solche Trennwandkolonne besitzt vorzugsweise 30 bis 120, besonders bevorzugt 45 bis 100 theoretische Trennstufen. Mit dieser Ausführung kann das erfindungsgemäße Verfahren besonders günstig durchgeführt werden.

Dabei weisen der obere gemeinsame Teilbereich 1 des Zulauf- und Entnahmeteils der Trennwandkolonne vorzugsweise 5 bis 50 %, besonders bevorzugt 15 bis 30 %, der Verstärkungsteil 2 des Zulaufteils vorzugsweise 5 bis 50 %, besonders bevorzugt 15 bis 30 %, der Abtriebsteil 4 des Zulaufteils vorzugsweise 5 bis 50. %, besonders bevorzugt 15 bis 30 %, der Abtriebsteil 3 des Entnahmeteils vorzugsweise 5 bis 50 %, besonders bevorzugt 15 bis 30 %, der Verstärkungsteil 5 des Entnahmeteils vorzugsweise 5 bis 50 %, besonders bevorzugt 15 bis 30 %, und der gemeinsame untere Teilbereich 6 des Zulauf- und Entnahmeteils der Kolonne vorzugsweise 5 bis 50 %, besonders bevorzugt 15 bis 30 % der Gesamtzahl der theoretischen Trennstufen der Kolonne auf.

Vorzugsweise beträgt die Summe der Zahl der theoretischen Trennstufen der Teilbereiche 2 und 4 im Zulaufteil 80 bis 110 %, besonders bevorzugt 90 bis 100 % der Summe der Zahl der Trennstufen der Teilbereiche 3 und 5 im Entnahmeteil.

Gleichfalls günstig ist es, die Zulaufstelle, über die der Zulauf Z in die Kolonne eingespeist wird, und die Seitenabzugsstelle, über die das Oxiran als Mittelsieder entnommen wird, hinsichtlich der Lage der theoretischen Trennstufen auf unterschiedlicher Höhe in der Kolonne anzuordnen. Vorzugsweise ist die Zulaufstelle um 1 bis 8, besonders bevorzugt um 3 bis 5 theoretische Trennstufen höher oder niedriger angeordnet als die Seitenabzugsstelle.

Die beim erfindungsgemäßen Verfahren verwendete Trennwandkolonne kann vorzugsweise sowohl als Packungskolonne mit Füllkörpern oder geordneten Packungen als auch als Bodenkolonne ausgeführt werden. Beispielsweise können als geordnete Packungen Blech- oder Gewebepackungen mit einer spezifischen Oberfläche von 100 bis 1000 m²/m³, bevorzugt etwa 250 bis 750 m²/m³ eingesetzt werden. Solche Packungen bieten eine hohe Trennleistung bei gleichzeitig niedrigem Druckverlust pro Trennstufe.

Vorzugsweise wird bei vorstehend genannter Ausführung der Kolonne der durch die Trennwand unterteilte Teilbereich der Kolonne, bestehend aus dem Verstärkungsteil 2 des Zulaufteils, dem Abtriebsteil 3 des Entnahmeteils, dem Abtriebsteil 4 des Zulaufteils und dem Verstärkungsteil 5 oder Teilen davon, mit geordneten Packungen oder Füllkörpern bestückt. Die Trennwand kann in diesen Teilbereichen wärmeisolierend ausgeführt werden.

Das Rohoxiran wird nun in Form des Zulaufstroms, der bezüglich des Oxirans Leicht- und Hochsieder enthält, über den Zulauf Z kontinuierlich in die Kolonne eingebracht. Dieser Zulaufstrom ist im Allgemeinen flüssig. Es kann jedoch von Vorteil sein, den Zulaufstrom einer Vorverdampfung zu unterziehen, und anschließend zweiphasig, d. h. gasförmig und flüssig oder in Form eines gasförmigen und eines flüssigen Stromes der Kolonne zuzuführen. Diese Vorverdampfung bietet sich besonders dann an, wenn der Zulaufstrom größere Mengen an Leichtsiedern enthält. Durch die Vorverdampfung kann der Abtriebsteil der Kolonne wesentlich entlastet werden.

Zweckmäßigerweise wird der Zulaufstrom mittels einer Pumpe oder über eine statische Zulaufhöhe von mindestens 1 m mengengeregelt in den Zulaufteil aufgegeben. Vorzugsweise erfolgt diese Zugabe über eine Kaskadenregelung in Verbindung mit der Flüssigkeitsstandregelung des Auffangraumes des Zulaufteils. Dabei wird die Regelung so eingestellt, dass die auf das Verstärkungsteil 2 aufgegebene Flüssigkeitsmenge nicht unter 30 % des Normalwertes sinken kann. Es hat sich gezeigt, dass eine derartige Vorgehensweise zur Kompensation von störenden Schwankungen bezüglich der Zulaufmenge oder der Zulaufkonzentration wichtig ist.

Ähnlich wichtig ist, dass die Aufteilung der aus dem Abtriebsteil 3 des Entnahmeteils der Kolonne ablaufenden Flüssigkeit auf den Seitenabzug und auf den Verstärkungsteil 5 des Entnahmeteils durch eine Regelung so eingestellt wird, dass die auf den Teilbereich 5 aufgegebene Flüssigkeitsmenge nicht unter 30 % des Normalwertes sinken kann.

Die Einhaltung dieser Voraussetzungen muss durch entsprechende Regelvorschriften sichergestellt werden.

Regelungsmechanismen zum Betreiben von Trennwandkolonnen wurden beispielsweise beschrieben in Chem. Eng. Technol. 10 (1987) 92-98, Chem.-Ing.-Technol. 61 (1989) Nr. 1, 16-25, Gas Separation and Purification 4 (1990) 109-114, Process Engineering 2 (1993) 33-34, Trans IChemE 72 (1994) Part A 639-644, Chemical Engineering 7 (1997) 72-76. Die bei diesem Stand der Technik angegebenen Regelungsmechanismen können auch für das erfindungsgemäße Verfahren angewendet bzw. auf dieses übertragen werden.

Für die kontinuierlich betriebene Reindestillation des Oxirans hat sich nachfolgend beschriebenes Regelungsprinzip als besonders günstig erwiesen. Es ist in der Lage, Lastschwankungen gut zu verkraften. Vorzugsweise erfolgt somit die Destillatentnahme temperaturgeregelt.

Im oberen Kolonnenteil 1 ist eine Temperaturregelung vorgesehen, die als Stellgröße die Ablaufmenge, das Rücklaufverhältnis oder bevorzugt die Rücklaufmenge nutzt. Dabei befindet sich die Messstelle für die Temperaturregelung vorzugsweise um 3 bis 8, besonders bevorzugt um 4 bis 6 theoretische Trennstufen unterhalb des oberen Endes der Kolonne.

Durch eine geeignete Temperatureinstellung wird dann die aus dem Kolonnenteil 1 ablaufende Flüssigkeit am oberen Ende der Trennwand so aufgeteilt, dass das Verhältnis des Flüssigkeitsstroms zum Zulaufteil zu dem zum Entnahmeteil vorzugsweise 0,1 bis 1,0, besonders bevorzugt 0,3 bis 0,6, beträgt.

Vorzugsweise wird bei dieser Methode die ablaufende Flüssigkeit in einem in oder außerhalb der Kolonne angeordneten Auffangraum gesammelt, woraus sie dann kontinuierlich in die Kolonne eingespeist wird. Dieser Auffangraum kann somit die Aufgabe einer Pumpenvorlage übernehmen oder für eine ausreichend hohe statische Flüssigkeitshöhe sorgen, die eine durch Stellorgane, beispielsweise Ventile, geregelte Flüssigkeitsweiterleitung ermöglicht. Bei der Verwendung von gepackten Kolonnen wird die Flüssigkeit zunächst in Sammler gefasst und von dort aus in einen innenliegenden oder außenliegenden Auffangraum geleitet.

Der Brüdenstrom am unteren Ende der Trennwand 7 wird durch die Wahl und/oder Dimensionierung der Trenneinbauten und/oder den Einbau druckmindemder Vorrichtungen, beispielsweise von Blenden, so eingestellt, dass das Verhältnis des Brüdenstroms im Zulaufteil zu dem des Entnahmeteils vorzugsweise 0,8 bis 1,2, bevorzugt 0,9 bis 1,1, beträgt.

Beim vorstehend genannten Regelprinzip ist des weiteren eine Temperaturregelung im unteren gemeinsamen Kolonnenteil 6 vorgesehen, die als Stellgröße die Sumpfentnahmemenge nutzt. Somit kann die Entnahme des Sumpfprodukts temperaturgeregelt erfolgen. Dabei ist die Messstelle für die Temperaturregelung vorzugsweise um 3 bis 6, besonders bevorzugt 4 bis 6 theoretische Trennstufen oberhalb des unteren Endes der Kolonne angeordnet.

Zusätzlich kann die genannte Standregelung am Kolonnenteil 6 und damit für den Kolonnensumpf als Stellgröße für die Seitenentnahmemenge genutzt werden. Hierzu wird als Regelgröße der Flüssigkeitsstand im Verdampfer verwendet.

Als Stellgröße der Heizleistung kann auch der Differenzdruck über die Kolonne genutzt werden. Günstigerweise wird die Destillation bei einem Kopfdruck zwischen 1 und 10 bar, bevorzugt zwischen 2 und 5 bar, durchgeführt. Dementsprechend wird zur Einhaltung dieses Druckbereiches die Heizleistung des Verdampfers am Kolonnenboden gewählt.

Die Destillation findet dann vorzugsweise in einem Temperaturbereich von 35 bis 110 °C, besonders bevorzugt 45 bis 90 °C statt. Die Destillationstemperatur wird dabei am Seitenabzug gemessen.

Für die Destillation von Rohpropylenoxid hat sich auch insbesondere ein Druck von ca. 3 bar und eine Temperatur am Seitenabzug von ca. 60 °C bewährt, wobei ein hochreines Propylenoxid erhalten wird.

Um die Trennwandkolonne störungsfrei betreiben zu können, werden die vorstehend genannten Regelinechanismen zumeist in Kombination angewendet.

Bei der Trennung von Mehrstoffgemischen in eine Leichtsieder-, Mittelsieder- und Hochsiederfraktion existieren üblicherweise Spezifikationen über den maximal zulässigen Anteil an Leichtsiedern und Hochsiedern in der Mittelfraktion. Hierbei werden entweder einzelne, für das Trennproblem kritische Komponenten, sogenannte Schlüsselkomponenten, oder die Summe von mehreren Schlüsselkomponenten spezifiziert.

Die Einhaltung der Spezifikation für die Hochsieder in der Mittelsiederfraktion wird vorzugsweise über das Aufteilungsverhältnis der Flüssigkeitsmenge am oberen Ende der Trennwand geregelt. Dabei wird das Aufteilungsverhältnis so eingestellt, dass die Konzentration an Schlüsselkomponenten für die Hochsiederfraktion in der Flüssigkeit am oberen Ende der Trennwand 10 bis 80 Gew.-%, bevorzugt 30 bis 50 Gew.-% des Wertes ausmacht, der im Seitenabzug erzielt werden soll. Die Flüssigkeitsaufteilung kann dann so eingestellt werden, dass bei höheren Gehalten an Schlüsselkomponenten der Hochsiederfraktion mehr und bei niedrigeren Gehalten an Schlüsselkomponenten weniger Flüssigkeit auf den Zulaufteil geleitet wird.

Entsprechend wird die Spezifikation für die Leichtsieder in der Mittelsiederfraktion durch die Heizleistung geregelt. Hierbei wird die Heizleistung im Verdampfer so eingestellt, dass die Konzentration an Schlüsselkomponenten für die Leichtsiederfraktion in der Flüssigkeit am unteren Ende der Trennwand 10 bis 80 Gew.-%, bevorzugt 30 bis 50 Gew.-% des Wertes ausmacht, der im Seitenabzugsprodukt erzielt werden soll. Somit wird die Heizleistung dahingehend eingestellt, dass bei höherem Gehalt an Schlüsselkomponenten der Leichtsiederfraktion die Heizleistung erhöht und bei niedrigerem Gehalt an Schlüsselkomponenten der Leichtsiederfraktion die Heizleistung verringert wird.

Die Konzentration von Leicht- und Hochsiedern in der Mittelsiederfraktion kann nach üblichen Analysemethoden ermittelt werden. Beispielsweise kann zur Detektion Infrarot-Spektroskopie verwendet werden, wobei die im Reaktionsgemisch vorliegenden Verbindungen an Hand ihrer charakteristischen Absorptionen identifiziert werden. Diese Messungen können inline direkt in der Kolonne vorgenommen werden. Bevorzugt werden jedoch gaschromatographische Methoden verwendet. Hierbei ist dann vorgesehen, dass das obere und untere Ende der Trennwand Probeentnahmemöglichkeiten aufweisen. Somit können aus der Kolonne kontinuierlich oder in zeitlichen Abständen flüssige oder gasförmige Proben entnommen und hinsichtlich ihrer Zusammensetzung untersucht werden. In Abhängigkeit von der Zusammensetzung kann dann auf die entsprechenden Regelmechanismen zurückgegriffen werden.

In einer speziellen Ausführung der Trennwandkolonne ist es auch möglich, Zulaufteil und Entnahmeteil, die durch die Trennwand 7 voneinander separiert sind, nicht in einer Kolonne zu vereinen, sondern räumlich voneinander zu trennen. Somit kann in dieser speziellen Ausführung die Trennwandkolonne auch aus mindestens zwei voneinander räumlich getrennten Kolonnen bestehen, die dann aber miteinander thermisch gekoppelt sein müssen. Solche thermisch miteinander gekoppelten Kolonnen tauschen miteinander Dampf und Flüssigkeit aus, wobei die Energiezufuhr aber nur über eine Kolonne erfolgt. Diese spezielle Ausführung bietet den Vorteil, dass die thermisch miteinander gekoppelten Kolonnen auch unter verschiedenen Drücken betrieben werden können, wobei eine noch bessere Einstellung des zur Destillation erforderlichen Temperaturniveaus möglich sein kann als bei der herkömmlichen Trennwandkolonne.

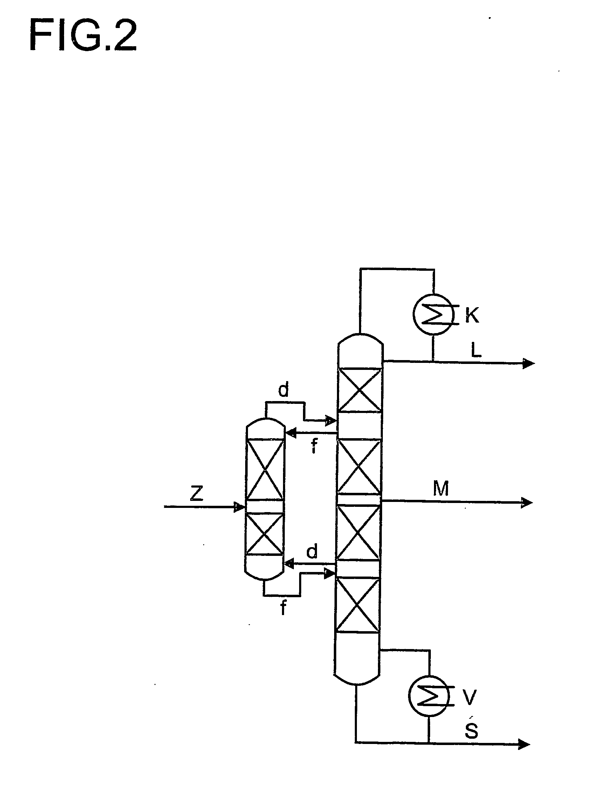

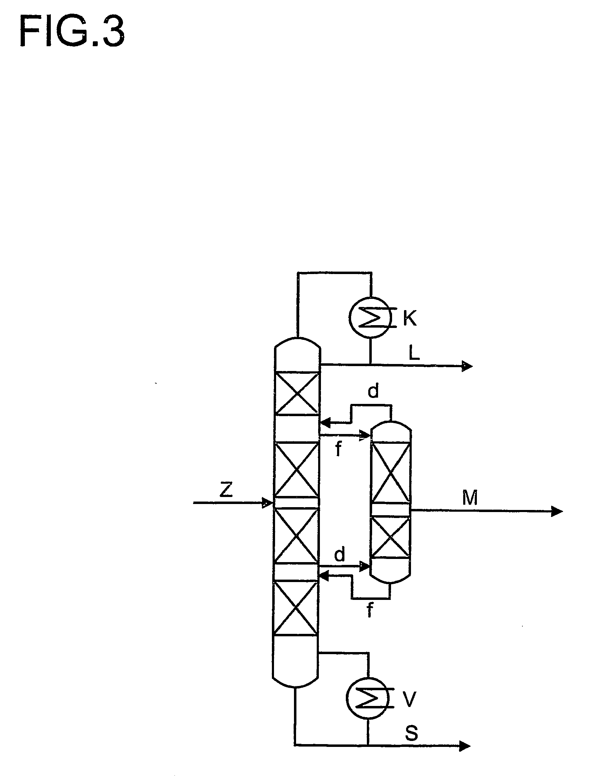

Beispiele für Trennwandkolonnen in der speziellen Ausführung der thermisch gekoppelten Kolonnen sind schematisch in Figur 2 und 3 dargestellt.

Figur 2 zeigt dabei eine Variante, in der die Energiezufuhr über den Verdampfer V der Kolonne erfolgt, die der Kolonne, die den Zulauf Z besitzt, über den das Rohoxiran eingespeist wird, nachgeschaltet ist. Dabei wird das Rohoxiran in der ersten Kolonne zunächst in eine Leicht- und Hochsiedersiederfraktion, die auch Mittelsieder enthält, aufgetrennt. Die resultierenden Fraktionen werden anschließend in die zweite Kolonne überführt, wobei die die Mittelsieder enthaltende Leichtsiederfraktion am oberen Ende und die die Mittelsieder enthaltende Hochsiederfraktion am unteren Ende der zweiten Kolonne eingespeist werden. Die Leichtsieder L werden über den Kopf der Kolonne abdestilliert und über den Kondensator K kondensiert. Die Hochsieder S werden mit dem Sumpf der Kolonne erhalten. Aus dem Seitenabzug für Mittelsieder M kann das hochreine Propylenoxid entnommen werden. Über d und f können beide Kolonnen Dampf und Flüssigkeit austauschen.

Figur 3 zeigt eine weitere Variante thermisch gekoppelter Kolonnen. In dieser Ausführungsform erfolgt die Energiezufuhr über den Verdampfer V der Kolonne, in die auch das Rohoxiran über den Zulauf Z eingespeist wird. Über den Kopf dieser Kolonne werden die Leichtsieder L abdestilliert und mit Hilfe des Kondensators K kondensiert. Mit dem Sumpf werden die Hochsieder S erhalten. Mit Mittelsieder angereicherte Leichtsieder L werden nun in den oberen Teil der nachgeschalteten Kolonne überführt, mit Mittelsieder angereicherte Hochsieder S in den unteren Teil der nachgeschalteten Kolonne. Aus dem Seitenabzug für Mittelsieder M kann das hochreine Propylenoxid entnommen werden. Über d und f können beide Kolonnen Dampf und Flüssigkeit austauschen.

Auch die Kolonnen nach Fig. 2 und 3 können als Packungskolonnen mit Füllkörpern oder geordneten Packungen oder als Bodenkolonnen ausgeführt werden. Beispielsweise können als geordnete Packungen Blech- oder Gewebepackungen mit einer spezifischen Oberfläche von 100 bis 1000 m²/m³, bevorzugt etwa 250 bis 750 m²/m³ eingesetzt werden. Solche Packungen bieten eine hohe Trennleistung bei gleichzeitig niedrigem Druckverlust pro Trennstufe.

Es ist ein Ziel des erfindungsgemäßen Verfahrens, Oxirane zu erhalten, in denen keine Verunreinigung in einer Konzentration von über 0,1 % vorliegt oder in denen die Summe aller Verunreinigungen nicht größer als 0,1 % ist. Insbesondere sollen Oxirane mit einer Reinheit von vorzugsweise mindestens 99,9 %, besonders bevorzugt jedoch mindestens 99,99 % erhalten werden. Die %-Angaben beziehen sich auf das Gewicht, wobei die Summe aus Oxiran und darin enthaltenen weiteren Komponenten 100 % beträgt.

Betrifft das erfindungsgemäße Verfahren die Reindestillation von Propylenoxid, so soll dieses in einer Reinheit von vorzugsweise mindestens 99,99 Gew.-% erhalten werden. Im Produkt soll dann die Konzentration der Schlüsselkomponenten in den Leichtsiedern (z. B. Acetaldehyd, Methylformiat) und der Schlüsselkomponenten in den Hochsiedern (z. B. Methanol, Wasser, Propylenglykol) vorzugsweise unter 0,01 Gew.-% liegen, wobei die Gesamtsumme 100 Gew.-% ergibt.

Für das erfindungsgemäße Verfahren zur kontinuierlich betriebenen Reindestillation des bei der vorzugsweise koppelproduktfreien Oxiransynthese entstehenden Oxirans in einer Trennwandkolonne können für die Oxiransynthese die aus dem Stand der Technik bekannten Edukte verwendet werden.

Bevorzugt werden organische Verbindungen umgesetzt, die mindestens eine C-C-Doppelbindung aufweisen. Als Beispiele für solche organischen Verbindungen mit mindestens einer C-C-Doppelbindung seien folgende Alkene genannt:
Ethen, Propylen, 1-Buten, 2-Buten, Isobuten, Butadien, Pentene, Piperylen, Hexene, Hexadiene, Heptene, Octene, Düsobuten, Trimethylpenten, Nonene, Dodecen, Tridecen, Tetra- bis Eicosene, Tri- und Tetrapropen, Polybutadiene, Polyisobutene, Isoprene, Terpene, Geraniol, Linalool, Linalylacetat, Methylencyclopropan, Cyclopenten, Cyclohexen, Norbornen, Cyclohepten, Vinylcyclohexan, Vinyloxiran, Vinylcyclohexen, Styrol, Cycloocten, Cyclooctadien, Vinylnorbornen, Inden, Tetrahydroinden, Methylstyrol, Dicyclopentadien, Divinylbenzol, Cyclododecen, Cyclododecatrien, Stilben, Diphenylbutadien, Vitamin A, Betacarotin, Vinylidenfluorid, Allylhalogenide, Crotylchlorid, Methallylchlorid, Dichlorbuten, Allylalkohol, Methallylalkohol, Butenole, Butendiole, Cyclopentendiole, Pentenole, Octadienole, Tridecenole, ungesättigte Steroide, Ethoxyethen, Isoeugenol, Anethol, ungesättigte Carbonsäuren wie z.B. Acrylsäure, Methacrylsäure, Crotonsäure, Maleinsäure, Vinylessigsäure, ungesättigte Fettsäuren, wie z.B. Ölsäure, Linolsäure, Palmitinsäure, natürlich vorkommende Fette und Öle.

Bevorzugt werden Alkene verwendet, die 2 bis 8 Kohlenstoffatome enthalten. Besonders bevorzugt werden Ethen, Propen und Buten umgesetzt. Insbesondere bevorzugt wird Propen umgesetzt.

Als Hydroperoxid können die bekannten Hydroperoxide, die für die Umsetzung der organischen Verbindung geeignet sind, eingesetzt werden. Beispiele für solche Hydroperoxide sind etwa tert-Butylhydroperoxid oder Ethylbenzolhydroperoxid. Bevorzugt wird als Hydroperoxid für die Oxiransynthese Wasserstoffperoxid eingesetzt, wobei auch eine wässerige Wasserstoffperoxidlösung verwendet werden kann.

Zur Herstellung von Wasserstoffperoxid kann dabei beispielsweise auf das Anthrachinonverfahren zurückgegriffen werden, nach dem praktisch die gesamte Menge des weltweit produzierten Wasserstoffperoxids hergestellt wird. Dieses Verfahren beruht auf der katalytischen Hydrierung einer Anthrachinon-Verbindung zur entsprechenden Anthrahydrochinon-Verbindung, nachfolgender Umsetzung derselben mit Sauerstoff unter Bildung von Wasserstoffperoxid und anschließender Abtrennung des gebildeten Wasserstoffperoxids durch Extraktion. Der Katalysezyklus wird durch erneute Hydrierung der rückgebildeten Anthrachinon-Verbindung geschlossen.

Einen Überblick über das Anthrachinonverfahren gibt "Ullmann's Encyclopedia of Industrial Chemistry", 5. Auflage, Band 13, Seiten 447 bis 456.

Ebenso ist es denkbar, zur Wasserstoffperoxidgewinnung Schwefelsäure durch anodische Oxidation unter gleichzeitiger kathodischer Wasserstoffentwicklung in Peroxodischwefelsäure zu überführen. Die Hydrolyse der Peroxodischwefelsäure führt dann auf dem Weg über Peroxoschwefelsäure zu Wasserstoffperoxid und Schwefelsäure, die damit zurückgewonnen wird.

Möglich ist selbstverständlich auch die Darstellung von Wasserstoffperoxid aus den Elementen.

Für die Oxiransynthese aus dem Hydroperoxid und der organischen Verbindung können zur größeren Effizienz der Umsetzung auch ein oder mehrere geeignete Katalysatoren zugesetzt werden, wobei wiederum heterogene Katalysatoren bevorzugt eingesetzt werden.

Es sind alle heterogenen Katalysatoren denkbar, die für die jeweilige Umsetzung geeignet sind. Bevorzugt werden dabei Katalysatoren verwendet, die ein poröses oxidisches Material, wie z. B. einen Zeolith, umfassen. Vorzugsweise werden Katalysatoren eingesetzt, die als poröses oxidisches Material einen Titan-, Germanium-, Tellur-, Vanadium-, Chrom-, Niob- oder Zirkonium-haltigen Zeolith umfassen.

Dabei sind im einzelnen Titan-, Germanium-, Tellur-, Vanadium-, Chrom-, Niob-, Zirkonium-haltige Zeolithe mit Pentasil-Zeolith-Struktur, insbesondere die Typen mit röntgenografischer Zuordnung zur ABW-, ACO-, AEI-, AEL-, AEN-, AET-, AFG-, AFI-, AFN-, AFO-, AFR-, AFS-, AFT-, AFX-, AFY-, AHT-, ANA-, APC-, APD-, AST-, ATN-, ATO-, ATS-, ATT-, ATV-, AWO-, AWW-, BEA-, BIK-, BOG-, BPH-, BRE-, CAN-, CAS-, CFI-, CGF-, CGS-, CHA-, CHI-, CLO-, CON-, CZP-, DAC-, DDR-, DFO-, DFT-, DOH-, DON-, EAB-, EDI-, EMT-, EPI-, ERI-, ESV-, EUO-, FAU-, FER-, GIS-, GME-, GOO-, HEU-, IFR-, ISV-, ITE-, JBW-, KFI-, LAU-, LEV-, LIO-, LOS-, LOV-, LTA-, LTL-, LTN-, MAZ-, MEI-, MEL-, MEP-, MER-, MFI-, MFS-, MON-, MOR-, MSO-, MTF-, MTN-, MTT-, MTW-, MWW-, NAT-, NES-, NON-, OFF-, OSI-, PAR-, PAU-, PHI-, RHO-, RON-, RSN-, RTE-, RTH-, RUT-, SAO-, SAT-, SBE-, SBS-, SBT-, SFF-, SGT-, SOD-, STF-, STI-, STT-, TER-, THO-, TON-, TSC-, VET-, VFI-, VNI-, VSV-, WIE-, WEN-, YUG-, ZON-Struktur sowie zu Mischstrukturen aus zwei oder mehr der vorgenannten Strukturen zu nennen. Denkbar sind für den Einsatz im erfindungsgemäßen Verfahren weiterhin titanhaltige Zeolithe mit der Struktur des ITQ-4, SSZ-24, TTM-1, UTD-1, CIT-1 oder CIT-5. Als weitere titanhaltige Zeolithe sind solche mit der Struktur des ZSM-48 oder ZSM-12 zu nennen.

Als besonders bevorzugt sind Ti-Zeolithe mit MFI-, MEL- oder MFI/MEL-Mischstruktur anzusehen. Ganz besonders bevorzugt sind im Einzelnen die Titanenthaltenden Zeolith-Katalysatoren, die im allgemeinen als "TS-1", "TS-2", "TS-3" bezeichnet werden, sowie Ti-Zeolithe mit einer zu β-Zeolith isomorphen Gerüststruktur.

Insbesondere ist es günstig, einen heterogenen Katalysator, der das titanhaltige Silikalit TS-1 umfasst, zu verwenden.

Dabei ist es auch möglich, als Katalysator das poröse oxidische Material an sich zu verwenden. Selbstverständlich ist es jedoch auch möglich, als Katalysator einen Formkörper einzusetzen, der das poröse oxidische Material umfasst. Dabei können zur Herstellung des Formkörpers, ausgehend von dem porösen oxidischen Material, alle Verfahren gemäß dem Stand der Technik eingesetzt werden.

Vor, während oder nach dem einen oder mehreren Formgebungsschritten in diesen Verfahren können auf das Katalysatormaterial Edelmetalle in Form geeigneter Edelmetallkomponenten, beispielsweise in Form von wasserlöslichen Salzen aufgebracht werden. Vorzugsweise wird dieses Verfahren angewendet, um Oxidationskatalysatoren auf der Basis von Titan- oder Vanadiumsilikaten mit Zeolithstruktur herzustellen, wobei Katalysatoren erhältlich sind, die einen Gehalt von 0,01 bis 30 Gew.-% an einem oder mehreren Edelmetallen aus der Gruppe Ruthenium, Rhodium, Palladium, Osmium, Iridium, Platin, Rhenium, Gold und Silber aufweisen. Derartige Katalysatoren sind beispielsweise in der DE-A 196 23 609.6 beschrieben.

Selbstverständlich können die Formkörper konfektioniert werden. Sämtliche Verfahren zur Zerkleinerung sind dabei denkbar, beispielsweise durch Splittung oder Brechen der Formkörper, ebenso wie weitere chemische Behandlungen, wie beispielsweise vorstehend beschrieben.

Bei Verwendung eines Formkörpers oder auch mehr davon als Katalysator kann dieser im erfindungsgemäßen Verfahren nach erfolgter Deaktivierung durch ein Verfahren regeneriert werden, bei dem die Regenerierung durch gezieltes Abbrennen der für die Deaktivierung verantwortlichen Beläge erfolgt. Dabei wird bevorzugt in einer Inertgasatmosphäre gearbeitet, die genau definierte Mengen an Sauerstoff-liefernden Substanzen enthält. Dieses Regenerierungsverfahren ist in der DE-A 197 23 949.8 beschrieben. Ferner können die dort bezüglich des Standes der Technik angegebenen Regenerierungsverfahren eingesetzt werden.

Als Lösungsmittel können vorzugsweise alle Lösungsmittel verwendet werden, die die in die Oxiransynthese eingesetzten Edukte ganz oder wenigstens teilweise lösen. Beispiele für Lösungsmittel sind aliphatische, cycloaliphatische und aromatische Kohlenwasserstoffe, Ester, Ether, Amide, Sulfoxide und Ketone sowie Alkohole. Die Lösungsmittel können dabei auch in Form von Mischungen eingesetzt werden. Bevorzugt werden Alkohole eingesetzt. Dabei ist der Einsatz von Methanol als Lösungsmittel besonders bevorzugt.

Als Reaktoren für die Oxiran-Synthese können selbstverständlich alle denkbaren, für die jeweiligen Reaktionen am besten geeigneten Reaktoren eingesetzt werden. Dabei ist für die Oxiran-Synthese ein Reaktor nicht auf einen einzelnen Behälter beschränkt. Vielmehr ist es auch möglich, beispielsweise eine Rührkesselkaskade einzusetzen.

Bevorzugt werden für die Oxiran-Synthese als Reaktoren Festbettreaktoren venvendet. Weiter bevorzugt werden als Festbettreaktoren Festbettrohrreaktoren eingesetzt.

Insbesondere wird für vorstehend beschriebene und bevorzugt verwendete Oxiransynthese als Reaktor für die Stufe (i) ein isothermer Festbettreaktor und für die Stufe (iii) ein adiabatischer Festbettreaktor verwendet.

Es ist möglich, auch mehrere organische Verbindungen mit dem Hydroperoxid umzusetzen. Ebenso ist es denkbar, zur Umsetzung mehrere Hydroperoxide zu verwenden. Werden beispielsweise zwei organische Verbindungen und/oder mehrere Hydroperoxide miteinander in den jeweiligen Stufen umgesetzt, so können in den Mischungen verschiedenartige Produkte, die aus den Umsetzungen resultieren, vorliegen. Jedoch können auch solche Mischungen aus zwei verschiedenen Oxiranen mit gutem Erfolg nach dem erfindungsgemäßen Verfahren unter Verwendung einer Trennwandkolonne mit zwei flüssigen Seitenabzügen abgetrennt werden, sofern die Siedepunkte nicht zu nahe beieinander liegen.

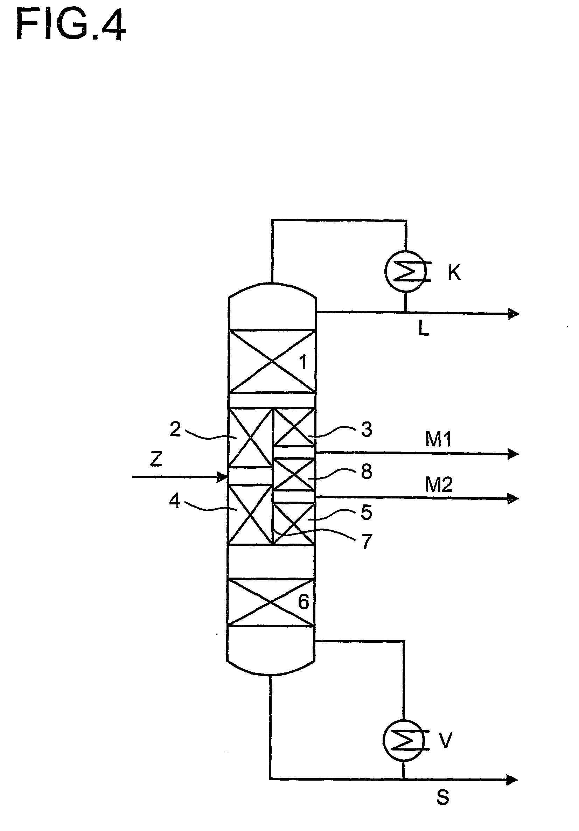

Eine Trennwandkolonne mit zwei Seitenabzügen ist in Figur 4 skizziert. Dabei werden aus den übereinander liegenden Seitenabzügen das niedriger siedende Oxiran an der oberen Seitenabzugsstelle M1 und das höher siedende Oxiran an der unteren Seitenabzugsstelle M2 entnommen. Bei dieser Anordnung weist der Bereich der thermischen Kopplung 8 vorzugsweise fünf bis fünfzig, besonders bevorzugt fünfzehn bis dreißig Prozent der Gesamtzahl der theoretischen Trennstufen der Kolonne auf.

In einer bevorzugten Ausführung der Oxiransynthese wird als Hydroperoxid Wasserstoffperoxid verwendet und die organische Verbindung während der Reaktion in Kontakt mit einem heterogenen Katalysator gebracht. Weiter bevorzugt ist dann auch, dass als organische Verbindung Propylen eingesetzt wird und das Oxiran Propylenoxid ist. Somit betrifft in einer besonders bevorzugten Ausführung das erfindungsgemäße Verfahren die kontinuierlich betriebene Zwischenabtrennung des bei der koppelproduktfreien Propylenoxidsynthese entstehenden Propylenoxids unter Verwendung einer Trennwandkolonne.

Weiterer Gegenstand der Erfindung ist auch eine Vorrichtung zur Durchführung eines kontinuierlich betriebenen Verfahrens zur Reindestillation des bei der Oxiransynthese durch Umsetzung eines Hydroperoxids mit einer organischen Verbindung entstehenden Oxirans, umfassend wenigstens einen Reaktor zur Herstellung des Oxirans und wenigstens eine Trennwandkolonne zur Reindestillation des Oxirans.

Eine bevorzugte Ausführung einer Vorrichtung zur Durchführung eines kontinuierlich betriebenen Verfahrens zur Reindestillation des bei der Oxiransynthese durch Umsetzung eines Hydroperoxids mit einer organischen Verbindung entstehenden Oxirans ist dadurch gekennzeichnet, dass die Vorrichtung zur Herstellung des Oxirans wenigstens einen isothermen und einen adiabatischen Reaktor für die Durchführung der Stufen (i) und (iii) sowie eine Abtrennapparatur für die Stufe (ii) und eine Trennwandkolonne zur Reindestillation des Oxirans umfasst.

Die Trennwandkolonne kann dabei aus mindestens zwei thermisch gekoppelten Kolonnen bestehen.

Die Erfindung soll nun durch folgendes Beispiel erläutert werden.

### Beispiel

Nach dem in der WO 00/07965 beschriebenen mehrstufigen Verfahren wurde ausgehend von Propylen und Wasserstoffperoxid Propylenoxid hergestellt. Das erhaltene Rohpropylenoxid hatte etwa folgende Zusammensetzung:
ca. 0,2 Gew.-% leichtsiedende Komponenten, umfassend die Schlüsselkomponenten Methylfomiat und Acetaldehyd,
ca. 99,0 Gew.-% Propylenoxid,
ca. 0,8 Gew.-% hochsiedende Komponenten, umfassend die Schlüsselkomponenten Methanol, Wasser und 1,2-Propylenglykol.

Ziel der Destillation war es, durch Reindestillation ein Propylenoxid der Reinheit von mindestens 99,99 % zu erhalten, d. h. mit einem Summenanteil an Schlüsselkomponenten von unter 0,01 Gew.-%. Dazu wurde das Gemisch mit Hilfe einer Trennwandkolonne mit 45 bis 100 theoretischen Böden bei einem Kopfdruck von 3 bar und einer Temperatur von 62 °C destilliert. Die Heizleistung der Sumpfverdampfer der Trennwandkolonne wurde so eingestellt, dass eine Summe der Konzentrationen dieser Komponenten im Seitenabzug resultierte, die kleiner als 0,01 Gew.-% war.

Der benötigte Energieinhalt der Destillation, der aus der Verdampferleistung und der pro Stunde durch die Kolonne durchgesetzten Stoffmenge berechnet wurde, wurde als Maß für die Effektivität der Trennung eingesetzt. Als Kolonnenverschaltungen wurden die in der Tabelle aufgeführten Anordnungen gewählt:

| Kolonnenverschaltung | Energiebedarf/(kg Propylenoxid /h) [kW/(kg Propylenoxid/h)] | Energieeinsparung [%] |
|---|---|---|
| Konventionelle Kolonne mit Seitenabzug | 1,95 | - |
| Reihenschaltung zweier konventioneller Kolonnen | 1,74 | 10,8 |
| Trennwandkolonne | 1,45 | 25,6 |

Es wird deutlich, dass die Trennwandverschaltung energetisch einen erheblichen Vorteil gegenüber den beiden konventionellen Destillationsanordnungen besaß. Zudem war die thermische Belastung des hochreaktiven und thermisch labilen Produkts geringer, da zur Trennung im Vergleich zu der konventionellen Kolonne mit Seitenabzug wesentlich geringere Rücklaufverhältnisse notwendig waren. Daher kam es zu einer geringeren Verweilzeit des Produkts in der Kolonne samt Verdampfer. Gegenüber der Reihenschaltung wurde das Produkt nur einmal in einem Verdampfer thermisch beansprucht.

### Bezugszeichenliste für Figuren 1, 2, 3 und 4:

- 1: gemeinsamer Teilbereich des Zulauf- und Entnahmeteils der Trennwandkolonne
- 2: Verstärkungsteil des Zulaufteils
- 3: Abtriebsteil des Entnahmeteils
- 4: Abtriebsteil des Zulaufteils
- 5: Verstärkungsteil des Entnahmeteils
- 6: gemeinsamer Teilbereich des Zulauf- und Entnahmeteils
- 7: Trennwand
- 8: Bereich der thermischen Kopplung

- Z: Zulauf
- L: Leichtsieder
- M: Seitenabzug für Mittelsieder
- M1: Seitenabzugsstelle für niedriger siedendes Oxiran
- M2: Seitenabzugsstelle für höher siedendes Oxiran
- S: Hochsieder
- K: Kondensator
- V: Verdampfer

- d: Dampf
- f: Flüssigkeit

Waagrechte und diagonale oder diagonal angedeutete Linien in den Kolonnen symbolisieren Packungen mit Füllkörpern oder geordnete Packungen, die in der Kolonne vorhanden sein können.

## Patentansprüche

1. Kontinuierlich betriebenes Verfahren zur Reindestillation des bei der Oxiransynthese durch Umsetzung eines Hydroperoxids mit einer organischen Verbindung entstehenden Oxirans, **dadurch gekennzeichnet, dass** das Roh-oxiran in einer Trennwandkolonne in eine Leicht-, Mittel- und Hochsiederfraktion aufgetrennt und als Mittelsieder am Seitenabzug entnommen wird und dass die Trennwandkolonne in Form thermisch gekoppelter Kolonnen ausgeführt ist.

2. Verfahren nach Anspruch 1, **dadurch gekennzeichnet, dass** die Trennwandkolonne 30 bis 120 theoretische Trennstufen besitzt.

3. Verfahren nach Anspruch 1 oder 2, **dadurch gekennzeichnet, dass** die Destillation bei einem Druck von 1 bis 10 bar und einer Temperatur von 35 bis 110 °C durchgeführt wird, wobei der Druck am Kopf der Kolonne und die Temperatur am Seitenabzug gemessen wird.

4. Verfahren nach einem der Ansprüche 1 bis 3, **dadurch gekennzeichnet, dass** im Oxiran keine Verunreinigung in einer Konzentration von über 0,1 Gew.-% vorliegt oder die Summe aller Verunreinigungen nicht größer als 0,1 Gew.-% ist.

5. Verfahren nach einem der Ansprüche 1 bis 4, **dadurch gekennzeichnet, dass** das Oxiran hergestellt wird durch ein Verfahren umfassend wenigstens die Stufen (i) bis (iii):
(i) Umsetzung des Hydroperoxids mit der organischen Verbindung unter Erhalt eines Produktgemisches, umfassend die umgesetzte organische Verbindung und nicht umgesetztes Hydroperoxid,
(ii) Abtrennung des nicht umgesetzten Hydroperoxids aus der aus Stufe (i) resultierenden Mischung,
(iii) Umsetzung des abgetrennten Hydroperoxids aus Stufe (ii) mit der organischen Verbindung.

6. Verfahren nach Anspruch 5, **dadurch gekennzeichnet, dass** in Stufe (i) ein isothermer Festbettreaktor, in Stufe (iii) ein adiabatischer Festbettreaktor und in Stufe (ii) eine Abtrennapparatur verwendet wird.

7. Verfahren nach einem der Ansprüche 1 bis 6, **dadurch gekennzeichnet, dass** als Hydroperoxid Wasserstoffperoxid und als organische Verbindung Propylen eingesetzt werden und die Umsetzung an einem heterogenen Katalysator unter Bildung von Propylenoxid als Oxiran abläuft.

8. Verfahren nach Anspruch 7, **dadurch gekennzeichnet, dass** als heterogener Katalysator der Zeolith TS-1 eingesetzt wird.

9. Vorrichtung zur Durchführung eines kontinuierlich betriebenen Verfahrens zur Reindestillation des bei der Oxiransynthese durch Umsetzung eines Hydroperoxids mit einer organischen Verbindung entstehenden Oxirans, **dadurch gekennzeichnet, dass** die Vorrichtung wenigstens einen isothermen und einen adiabatischen Reaktor sowie eine Abtrennapparatur zur Herstellung des Oxirans wie in Anspruch 7 definiert und eine Trennwandkolonne in Form thermisch gekoppelten Kolonnen zur Reindestillation des Oxirans umfasst.

## Claims

1. A continuously operated process for the purification by distillation of the oxirane formed in the oxirane synthesis by reaction of a hydroperoxide with an organic compound, wherein the crude oxirane is separated in a dividing wall column into low-, intermediate- and high-boiling fractions and the oxirane is taken off as intermediate boiler at the side offtake and the dividing wall column is embodied as thermally coupled columns.

2. The process according to claim 1 wherein the dividing wall column has from 30 to 120 theoretical plates.

3. The process according to claim 1 or 2 wherein the distillation is carried out at from 35 to 110°C and a pressure of from 1 to 10 bar, with the temperature being measured at the side offtake and the pressure being measured at the top of the column.

4. The process according to any of claims 1 to 3 wherein no impurity is present in the oxirane in a concentration of above 0.1% by weight or the sum of all impurities is no greater than 0.1 % by weight.

5. The process according to any of claims 1 to 4 wherein the oxirane is prepared by a process comprising at least the steps (i) to (iii):
(i) reaction of the hydroperoxide with the organic compound to give a product mixture comprising the unreacted organic compound and unreacted hydroperoxide,
(ii) separation of the unreacted hydroperoxide from the mixture resulting from step (i),
(iii) reaction of the hydroperoxide which has been separated off in step (ii) with the organic compound.

6. The process according to claim 5 wherein an isothermal fixed-bed reactor is used in step (i), an adiabatic fixed-bed reactor is used in step (iii) and a separation apparatus is used in step (ii).

7. The process according to any of claims 1 to 6 wherein the hydroperoxide used is hydrogen peroxide and the organic compound used is propylene and the reaction occurs over a heterogeneous catalyst to form propylene oxide as oxirane.

8. The process according to claim 7 wherein the heterogeneous catalyst used is the zeolite TS-1.

9. An apparatus for carrying out a continuously operated process for the purification by distillation of the oxirane formed in the oxirane synthesis by reaction of a hydroperoxide with an organic compound, which comprises at least one isothermal reactor and one adiabatic reactor and also a separation apparatus for preparing the oxirane as defined in claim 7 and a dividing wall column in the form of thermally coupled columns for purifying the oxirane by distillation.

## Revendications

1. Procédé réalisé en continu de distillation pure de l'oxirane issu de la synthèse d'oxirane par réaction d'un hydroperoxyde avec un composé organique, **caractérisé en ce que** l'oxirane brut est séparé dans une colonne à membrane en une fraction légère, une fraction intermédiaire et une fraction lourde, et est prélevé dans l'état intermédiaire au niveau d'un évent latéral, et **en ce que** la colonne à membrane est réalisée sous la forme de colonnes couplées thermiquement.

2. Procédé selon la revendication 1, **caractérisé en ce que** la colonne à membrane possède 30 à 120 étages de partition théoriques.

3. Procédé selon la revendication 1 ou 2, **caractérisé en ce que** la distillation est réalisée sous une pression de 1 à 10 bars, et à une température de 35 à 110°C, et dans lequel la pression est mesurée à la tête de la colonne et la température est mesurée au niveau de l'évent latéral.

4. Procédé selon l'une quelconque des revendications 1 à 3, **caractérisé en ce qu'**aucune impureté n'est présente dans l'oxirane à une concentration supérieure à 0,1 % en poids, ou **en ce que** la somme de toutes les impuretés n'est pas supérieure à 0,1 % en poids.

5. Procédé selon l'une quelconque des revendications 1 à 4, **caractérisé en ce que** l'oxirane est produit par un procédé impliquant au moins les étapes (i) à (iii) consistant à :
(i) faire réagir de l'hydroperoxyde avec le composé organique, en obtenant un mélange produit contenant le composé organique transformé et de l'hydroperoxyde non transformé,
(ii) séparer l'hydroperoxyde non transformé du mélange résultant de l'étape (i),
(iii) faire réagir l'hydroperoxyde séparé à l'étape (ii) avec le composé organique.

6. Procédé selon la revendication 5, **caractérisé en ce qu'**à l'étape (i), un réacteur à lit fixe isotherme est utilisé, à l'étape (iii), un réacteur à lit fixe adiabatique est utilisé, et à l'étape (ii), un appareil de séparation est utilisé.

7. Procédé selon l'une quelconque des revendications 1 à 6, **caractérisé en ce que** du peroxyde d'hydrogène est utilisé en tant qu'hydroperoxyde, et du propylène en tant que composé organique, et **en ce que** la réaction se déroule sur un catalyseur hétérogène en formant de l'oxyde de propylène en tant qu'oxirane.

8. Procédé selon la revendication 7, **caractérisé en ce que** de la zéolithe TS-1 est utilisée en tant que catalyseur hétérogène.

9. Dispositif pour la mise en oeuvre d'un procédé réalisé en continu de distillation pure de l'oxirane issu de la synthèse d'oxirane par réaction d'un hydroperoxyde avec un composé organique, **caractérisé en ce que** le dispositif fait intervenir dans la production de l'oxirane, telle que définie dans la revendication 7, au moins un réacteur isotherme et un réacteur adiabatique, ainsi qu'un appareil de séparation, et, pour la distillation pure de l'oxirane, une colonne à membrane sous la forme de colonnes couplées thermiquement.
